(19) Europäisches Patentamt / European Patent Office / Office européen des brevets

(11) **EP 3 778 553 A1**

(12) **EUROPEAN PATENT APPLICATION**
published in accordance with Art. 153(4) EPC

(43) Date of publication:
**17.02.2021 Bulletin 2021/07**

(21) Application number: **18914207.8**

(22) Date of filing: **30.07.2018**

(51) Int Cl.:
***C07C 213/06*** *(2006.01)* ***C07C 215/06*** *(2006.01)*
***C07C 215/10*** *(2006.01)* ***C07C 215/12*** *(2006.01)*
***C07C 215/16*** *(2006.01)* ***C07C 217/08*** *(2006.01)*
***C07C 217/76*** *(2006.01)*

(86) International application number:
**PCT/CN2018/097739**

(87) International publication number:
**WO 2019/196263 (17.10.2019 Gazette 2019/42)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **12.04.2018 CN 201810325842**

(71) Applicant: **Jiangsu Ruike Medical Science And Technology Co., Ltd.**
**Jiangsu 224100 (CN)**

(72) Inventors:
- **GAO, Zhaobo**
  **Taizhou, Zhejiang 318000 (CN)**
- **CHEN, Jianhua**
  **Taizhou, Zhejiang 318000 (CN)**
- **WAN, Zhidong**
  **Taizhou, Zhejiang 318000 (CN)**
- **HE, Dawei**
  **Taizhou, Zhejiang 318000 (CN)**
- **ZHOU, Zengle**
  **Taizhou, Zhejiang 318000 (CN)**
- **MA, Xiaodong**
  **Taizhou, Zhejiang 318000 (CN)**
- **XIANG, Wei**
  **Taizhou, Zhejiang 318000 (CN)**
- **LIN, Jingxin**
  **Taizhou, Zhejiang 318000 (CN)**
- **MEI, Yijiang**
  **Taizhou, Zhejiang 318000 (CN)**

(74) Representative: **Kador & Partner PartG mbB**
**Corneliusstraße 15**
**80469 München (DE)**

(54) **METHOD FOR PREPARING HEXAHYDROFURO-FURANOL DERIVATIVE, INTERMEDIATE THEREOF AND PREPARATION METHOD THEREFOR**

(57) The invention relates to the field of pharmaceutical synthesis, in particular to the preparation method of hexahydrofuro-furanol derivative, intermediates thereof and preparation methods thereof. The preparation methods comprises the steps of halogenation reaction, acylation reaction, enzymatic reduction reaction, reaction with amine compounds, reduction ring closure reaction (A1, A2, B, Cp1, $C_L$, Cf)

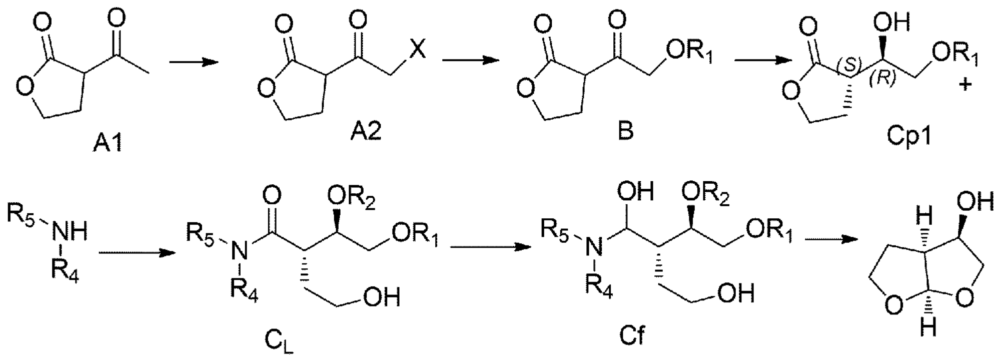

A1 A2 B Cp1 + $C_L$ Cf

,

EP 3 778 553 A1

wherein, $R_1$, $R_2$, $R_3$ are hydrogen or hydroxy protecting groups; $R_4$ and $R_5$ are the same or different and are phenyl, alkyl or substituted phenyl. In the preparation process of hexahydrofuro-furanol derivatives, the chirality is constructed by enzymatic method, and the product can be prepared with very high optical purity by adopting such technical means. The preparation method can be used to prepare the key intermediate, (3R, 3aS, 6aR)-hexahydrofuro[2,3-b]-3-ol, of Darunavir, in commercial production, which is a very economical route suitable for industrial production.

## Description

This application claims the priority of the Chinese patent application with the application number 201810325842.2 and the invention title of " METHOD FOR PREPARING HEXAHYDROFURO-FURANOL DERIVATIVE, INTERMEDIATE THEREOF AND PREPARATION METHOD THEREOF", filed to the National Intellectual Property Administration, PRC on April 12, 2018, , which is hereby incorporated herein by reference in its entirety.

## TECHNICAL FIELD

The invention relates to the field of pharmaceutical synthesis, in particular to the method for preparing hexahydrofuro-furanol derivative, the intermediate thereof and preparation method thereof.

## TECHNICAL BACKGROUND

The chemical name of the compound with the following formula Z structure is (3R, 3aS, 6aR)-hexahydrofuro[2,3-b]-3-ol:

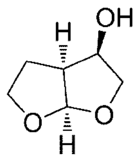

Z ,

which belongs to one of the hexahydrofuro-furanol derivatives and is an intermediate of the anti-AIDS drug Darunavir.

The Chinese patent applications No. 02817639.1 (application date: 2002-9-6) and 200580010400.X of Tibotec Pharmaceutical Co., Ltd., the original manufacturer of Darunavir, provide the preparation method of the above (3R, 3aS, 6aR)-hexahydrofuro[2,3-b]-3-ol, in which the raw material is the following compound of formula (3),

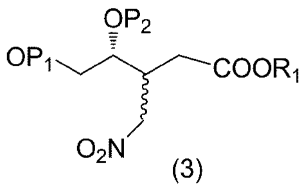

(3) ,

the compound of formula (3) is prepared from the starting material, the compound of formula (1).

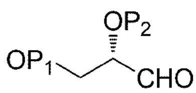

(1) ,

The Chinese patent application No. 2003080109926.4 of Sumitomo Chemical Co., Ltd., a generic drug manufacturer of Darunavir, provides the preparation method of the above (3R, 3aS, 6aR)-hexahydrofuro[2,3-b]-3-ol, in which the starting material is the following compound of formula VIII,

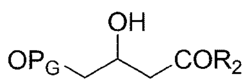

VIII .

Sumitomo Chemical Co., Ltd. of Japan chose to use chiral ligand catalysts for the generation of chiral conformation. Although this is indeed a method for constructing chiral conformation, it is not suitable for industrialization.

European patent application EP2634180A1 (application date: 2012-1-3) of Lonza Ltd. of Switzerland, a generic drug manufacturer of Darunavir, involves using carbonyl reductase polypeptides or microorganisms containing carbonyl reductase polypeptides to achieve the reduction of carbonyl groups to hydroxyl groups so as to construct the chirality of intermediate of Darunavir, and many kinds of commercially available enzymes such as Saccharomyces cerevisiae YNL331C are listed in the specification, and it is mentioned that the compound represented by the following formula Ia is a more suitable configuration,

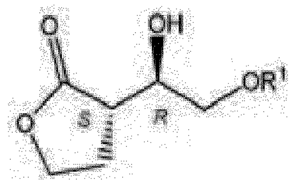

Ia

Considering that (3R,3aS,6aR)-hexahydrofuro[2,3-b]-3-ol is a key intermediate for the preparation of Darunavir drugs, it is necessary to develop more preparation methods for this key intermediate, which can not only obtain the key intermediate with high yield and high DE value, but also has low cost and mild reaction conditions, and is suitable for industrialization.

## SUMMARY

The method for preparing (3R, 3aS, 6aR)-hexahydrofuro[2,3-b]-3-ol of the invention starts from the choice of starting materials and the construction of chiral configuration, and a method for preparing the key intermediate of Darunavir with the starting materials different from that in the above-mentioned prior patent applications has been developed. The preparation method of the invention adopts an enzymatic method to construct chirality, which has low cost and mild reaction conditions, and provides an alternative route suitable for industrialization for the preparation of the key intermediate of Darunavir.

To achieve the technical objective of the present invention, the present invention provides the following technical solutions:

The first aspect of the present invention provides an intermediate compound for preparing (3R, 3aS, 6aR)-hexahydrofuro[2,3-b]-3-ol, having the following structural formula:

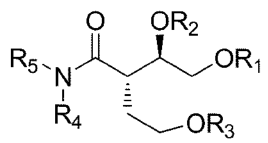

C ,

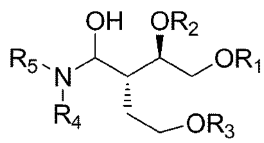

Cf ,

wherein, $R_1$, $R_2$, $R_3$ are hydrogen or hydroxy protecting groups; $R_4$, $R_5$ are the same or different, are phenyl, alkyl or substituted phenyl. The hydroxy protecting group is alkyl, silyl, $C_{2-11}$ acyl, $C_{4-9}$ cycloalkenyl, aryl, aralkyl, aroyl, phenyl, substituted phenyl; the silyl is tetramethylsilyl, trimethylsilyl, triethylsilyl, tri-n-butylsilyl, tert-butyldimethylsilyl; the alkyl is a $C_1$-$C_8$ alkyl; the aryl group is phenyl, furanyl, thienyl or indolyl; the substituted phenyl is alkyl-substituted phenyl, alkoxyalkyl-substituted phenyl, nitroalkyl-substituted phenyl or halogen-substituted phenyl; the alkyl-substituted phenyl is benzyl, benzhydryl, trityl; the alkoxyalkyl-substituted phenyl is p-methoxybenzyl; the nitroalkyl-substituted phenyl is p-nitrobenzyl; the halogen-substituted phenyl is p-chlorophenyl.

The second aspect of the present invention provides a method for preparing the above intermediate compound, which is prepared by reacting a compound of formula Cp with an amine compound,

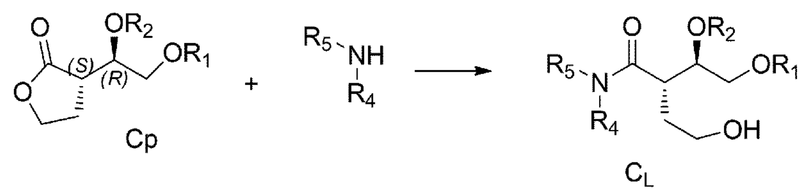

Cp + → $C_L$ ,

wherein, the definitions of $R_1$, $R_2$, $R_4$ and $R_5$ are the same as above.

More preferably, it is prepared by reacting a compound of formula Cp with an N-methylaniline compound,

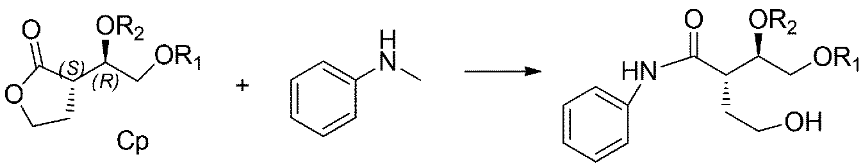

Cp C-1

wherein, the definitions of $R_1$, $R_2$ are the same as above.

It is prepared by enzymatic reduction reaction of compound of formula B to construct chirality,

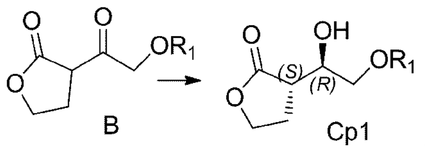

B Cp1 , ,

wherein, the definition of $R_1$ is the same as above.

The enzyme is a biological enzyme, such as aldo-keto reductase, wherein the aldo-keto reductase is derived from the Saccharomyces kudriavzevii strain. Its amino acid sequence is the protein shown in SEQ ID NO: 1, or the protein having aldo-keto reductase activity obtained from SEQ ID NO: 1 after substitution, deletion or addition of one or more amino acid residues, or the protein having 80% homology with the amino acid sequence shown in SEQ ID NO:1 and having aldo-keto reductase activity; its nucleotide sequence is shown as SEQ ID NO: 2 in the sequence listing. The aldo-keto reductase may be derived from whole cells of genetically engineered bacteria, broken enzyme liquid, lyophilized powder or immobilized enzymes or immobilized cells.

The amount of the enzyme fed may be in the range of 50-100 g/L, and the reaction temperature may be in the range of 25-37°C.

A coenzyme may be selectively added to the enzymatic reduction reaction, and the coenzyme is NADP+ or NADPH.

A glucose dehydrogenase may be selectively added to the enzymatic reduction reaction.

The enzymatic reduction reaction is carried out in the presence of a solvent which is a mixed solvent that is composed of water or buffer solution and an organic solvent.

The buffer solution is selected from one or more of phosphate buffer solution, carbonate buffer solution, Tri-HCl buffer solution, citrate buffer solution or MOPS buffer solution.

The organic solvent is selected from one or more of DMSO, ethyl acetate, butyl acetate, isopropanol, DMF, TBME, dichloromethane, and vinyl acetate.

In the biological conversion process of the enzymatic reduction reaction of the present invention, HPLC-MS and HPLC are used for monitoring until the substrate is fully utilized.

After the above enzymatic reduction reaction, a protecting group can be further introduced to prepare the compound of formula Cp,

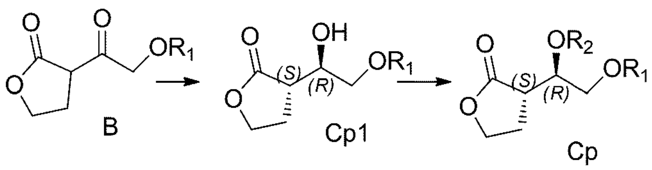

B Cp1 Cp ,

wherein, the definition of $R_1$ is the same as above, $R_2$ is a hydroxy protecting group, and the enzyme is the same as above.

Wherein, the compound of formula B is prepared from the compound of formula A2 by acylation, the reaction formula is as follows:

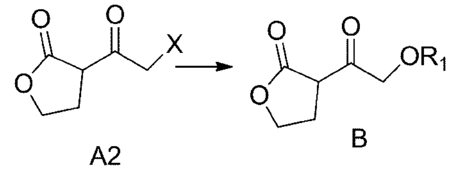

A2 B ,

wherein, X is halogen, and the definition of $R_1$ is the same as above.

The above compound of formula A2 is prepared from the compound of formula A1 by halogenation reaction, the reaction formula is as follows:

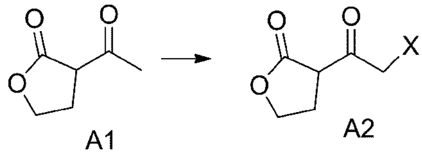

A1 A2 ,

wherein, X is halogen

The invention further provides a method for preparing the intermediate of (3R, 3aS, 6aR)-hexahydrofuro[2,3-b]-3-ol, compound of formula C-i, which is prepared from the compound of formula C-1 by deprotection reaction,

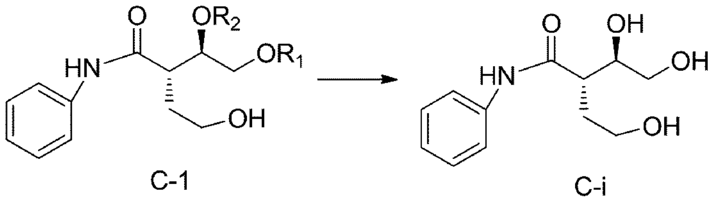

C-1 C-i ,

wherein, the definitions of $R_1$, $R_2$ are the same as above.

The third aspect of the present invention provides a method for preparing the intermediate of (3R, 3aS, 6aR)-hexahydrofuro[2,3-b]-3-ol, compound of formula Cf, which is prepared from a compound of formula $C_L$ by reduction reaction,

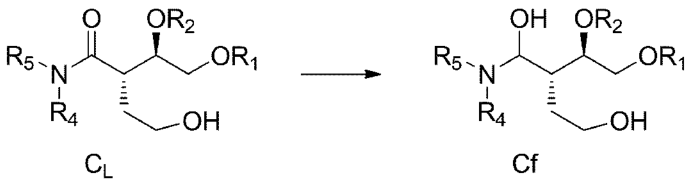

$C_L$ Cf ,

wherein, the definitions of $R_1$, $R_2$, $R_4$ and $R_5$ are the same as above.

More preferably, it is prepared from a compound of formula C-i by reduction reaction,

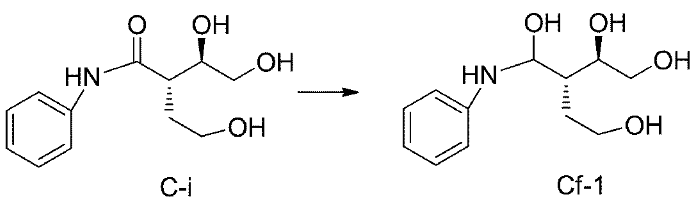

C-i Cf-1 .

In the reduction reaction, the reducing agent may be a boron reducing agent, an aluminum reducing agent or a silicon-lithium reducing agent. Such as sodium borohydride, sodium cyanoborohydride, lithium aluminum hydride, Red-Al, lithium aluminum hydride, diisobutyl aluminum hydride, lithium diisopropylamide, lithium hexamethyldisilazide may be used.

Before the above reduction reaction, a deprotection reaction can be carried out first, the reaction formula is as

follows:

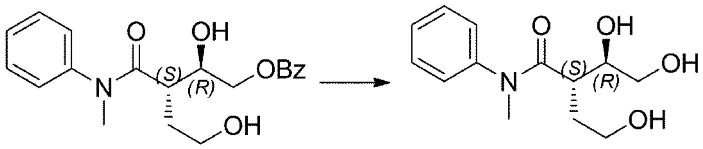

Cp2 Cp3

.

Alternatively, a cyclization reaction can be carried out first, and the reaction formula is as follows:

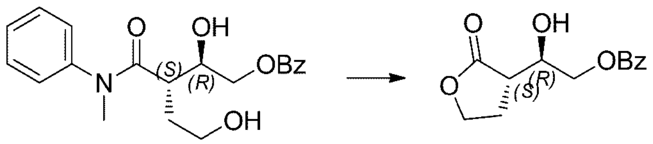

Cp2 Cp4

.

The prepared compound of formula Cp3 and compound of formula Cp4 are subjected to the carbonyl reduction reaction according to the above method, and the reducing reagent is the same as above.

That is, the present invention provides an alternative method for preparing the intermediate of (3R,3aS,6aR)-hexahydrofuro[2,3-b]-3-ol, compound of formula Cp, which is prepared from a compound of formula $C_L$ by ring closure reaction,

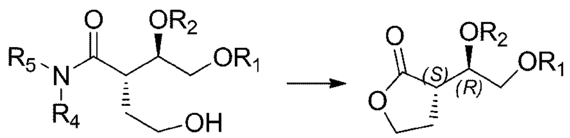

$C_L$ Cp

,

wherein, the definitions of $R_1$, $R_2$, $R_4$, $R_5$ are the same as above.

The fourth aspect of the present invention provides a method for preparing (3R, 3aS, 6aR)-hexahydrofuro[2,3-b]-3-ol, which is prepared from a compound of formula Cf by ring closure reaction,

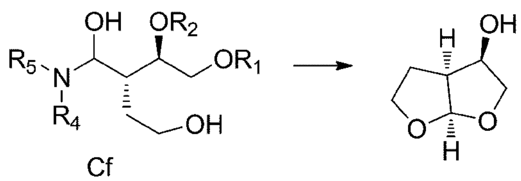

Cf

,

wherein, the definitions of $R_1$, $R_2$, $R_4$, $R_5$ are the same as above.

More preferably, it is prepared from a compound of formula Cf-1 by ring closure reaction,

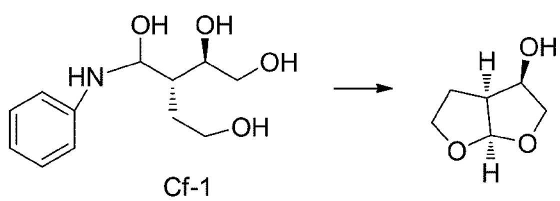

Cf-1

.

The reagent for the ring closure reaction is an acid or a base common in the art.

The above method for preparing (3R,3aS,6aR)-hexahydrofuro[2,3-b]-3-ol of the present invention includes the steps of halogenation reaction, acylation reaction, enzymatic reduction reaction, reaction with amine compounds, reductive ring closure reaction, and the reaction formula is:

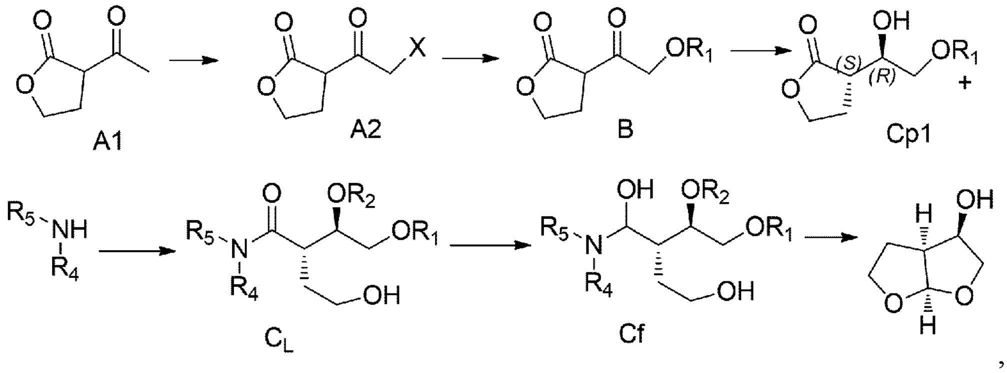

A1, A2, B, Cp1, $C_L$, Cf

wherein, X is halogen; the definitions of $R_1$, $R_2$, $R_4$, $R_5$ are the same as above.

More preferably, it is prepared by halogenation reaction, acylation reaction, enzymatic reduction reaction, reaction with amine compounds, deprotection reaction, reductive ring closure reaction

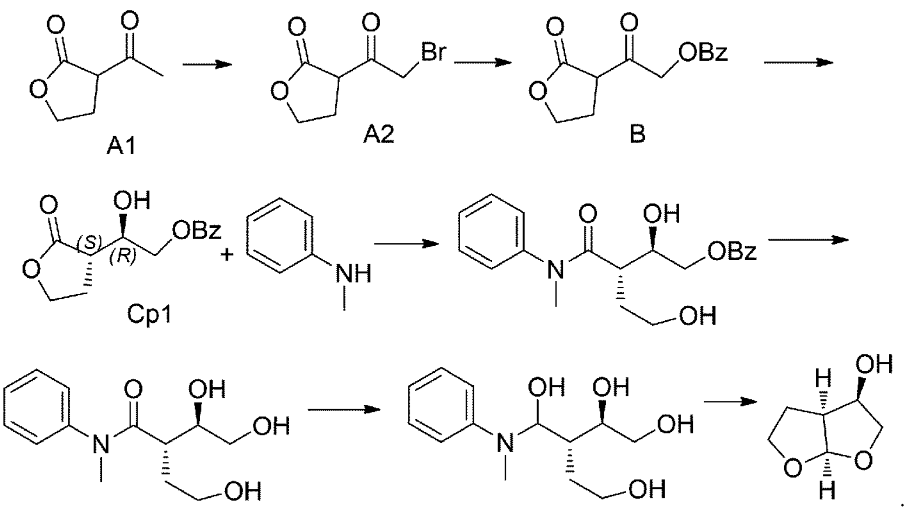

A1, A2, B, Cp1

Wherein, the method for constructing chirality by the reduction reaction is an enzymatic method, and the definition of the enzyme is the same as the above.

The preparation method of (3R,3aS,6aR)-hexahydrofuro[2,3-b]-3-ol provided by the present invention adopts enzymatic method to construct chirality, and the product can be prepared with high yield and high optical purity. Although the prior art such as the above European application has disclosed that the reduction of carbonyl to hydroxyl is achieved by carbonyl reductase polypeptides or microorganisms containing carbonyl reductase polypeptides, the enzyme used in the present invention has more significant advantages, shown in the higher optical purity of the obtained products and more suitable reaction conditions. The preparation method of (3R,3aS,6aR)-hexahydrofuro[2,3-b]-3-ol of the present invention is suitable for industrial production.

## EMBODIMENTS

In order to further understand the present invention, the preparation method of the hexahydrofuro-furanol derivatives provided by the present invention, the intermediates thereof and the preparation method thereof will be described in detail in combination with embodiments below. It should be understood that the description of these embodiments is only for further describing the features of the present invention in details, rather than limiting the scope of the present invention or the claims of the present invention.

Example 1:

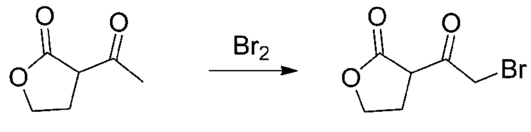


A1

The compound of formula A1 and dichloromethane were added to a reaction flask, cooled down, bromine was weighed, and dichloromethane was added for dilution, the diluted bromine was transferred to a funnel and slowly added dropwise, and the internal temperature was controlled. After dropwise addition, the solution was continued reacting with the temperature kept and the internal temperature was controlled. Water was added, the temperature was controlled, and the solution was allowd to stand, the liquid separation was performed. The lower organic phase was put into another reaction flask, water was added for extraction, and the liquid separation was performed. The lower organic phase was put into another reaction flask, 5% $NaHCO_3$ aqueous solution was added for extraction, and the liquid separation was performed. The lower organic phase was put into another reaction flask, and the upper aqueous phases were combined and extracted by adding dichloromethane, and the liquid separation was performed. The water phase was discard, and the organic phases were combined, water was added for extraction, and the liquid separation was performed, the water phase was discard, and the lower organic phase was placed in a rotary evaporator to concentrate until no solvent flows out, the yield was 90-95%.

Example 2:

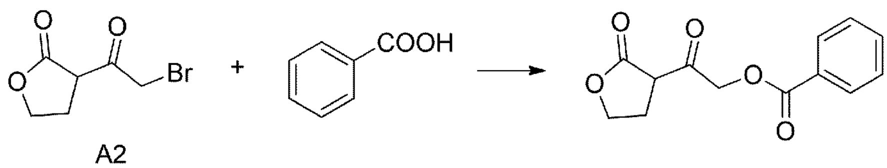

A2

Acetone, compound of formula A2 (X is bromine), benzoic acid were put into a reaction flask, the mixture was stirred and cooled down. Triethylamine was added to a tank and started to drop slowly, and the internal temperature was controlled. After dropwise addition, the mixture was raised to room temperature, and stirred for reaction. After the reaction was completed, suction filtration was performed, after that, the filtrate was transferred to a distillation flask to distill under reduced pressure and the temperature was controlled at 50-60°C until a pasty solid appeared in the distilling flask, then ethyl acetate was added. Ethyl acetate was added to the distilling flask and stirred until the pasty solid was dissolved, the material liquid in the distilling flask was transferred to the reaction flask. Saturated salt water was added to the reaction flask to wash, the solution was allowed to stand and the liquid separation was performed, water layers were combined, ethyl acetate was added for extraction, the solution was allowed to stand to separate into layers, the water layer was discarded, the organic layers were combined, anhydrous sodium sulfate was added to the organic layer and stirred to dryness, and suction filtration was performed. The filtrate was transferred to a reaction flask to distill under reduced pressure and the temperature was controlled at 50-60°C until a pasty solid appeared in the reaction solution, part of ethyl acetate was added, stirred and refluxed until dissolved to clear, the temperature was controlled at 50-60 °C, n-heptane was added to the tank, and was added slowly dropwise into the reaction bottle until completely added into the bottle. After slowly cooling down, the reaction solution was stirred with the temperature kept, suction filtration was performed and the target solid crude product was dried, the yield was 70-75%.

Example 3:

Preparation of whole cells of genetically engineered bacteria of aldo-keto reductase

The specific preparation method of genetically engineered bacteria of recombinant aldo-keto reductase was: selecting the gene sequence of aldo-keto reductase derived from Saccharomyces kudriavzevii, designing it artificially,

and synthesizing the artificially designed sequence by whole genes (synthesized by Genscript Biotechnology Co., Ltd.), cloning it into the Nde I and Xho I enzyme digestion sites of the expression vector pET28a, and transforming host E.coli BL21 (DE3) competent cells; picking the positive transformants and sequencing and identifying to obtain the recombinant expression vector; transforming the recombinant expression vector into E. coli BL21 (DE3) strain to obtain the genetically engineered bacteria of recombinant aldo-keto reductase which can induce the expression of recombinant aldo-keto reductase.

The genetically engineered bacteria of recombinant aldo-keto reductase were inoculated into LB medium containing kanamycin, and cultured at 37°C overnight to obtain seed culture solution; the seed culture solution was inoculated into TB medium containing kanamycin, and the inoculation volume was 1% of the volume of TB medium containing kanamycin; then it was incubated at 37°C for 2-5h. Sterile IPTG was added for inducing and the final concentration of IPTG was 0.1 mM, and the inoculum was continued to be incubated at 25°C for 20h. Finally, whole cells of the genetically engineered bacteria of aldo-keto reductase derived from Saccharomyces kudriavzevii were obtained by high-speed centrifugation. The whole cells of the genetically engineered bacteria were ultrasonically broken by ultrasonic method to obtain the broken enzyme solution of the whole cells of the genetically engineered bacteria of aldo-keto reductase derived from Saccharomyces kudriavzevii. The aldo-keto reductase is a protein whose amino acid sequence is shown in SEQ ID NO: 1, and the nucleotide sequence of the aldo-keto reductase gene is shown as SEQ ID NO: 2 in the sequence table.

After induced expression, there was a clear protein band at 45kDa, indicating that the aldo-keto reductase was highly expressed in the recombinant bacteria.

The enzyme activity of the pure protein of aldo-keto reductase was measured, the reaction system was at a volume of 0.25ml, including Tris-HCl, pH of 8.0, 2 mmol/L NADPH, 0.1 mmol/L substrate

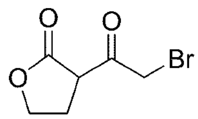

and appropriate amount of enzyme, the decrease of absorbance at 340nm was measured. The enzyme activity unit (U) was defined as the amount of enzyme required to catalyze the oxidation of 1umol NADPH per minute under the above conditions.

The results showed that the recombinant genetically engineered aldo-keto reductase has an activity of more than 20% higher than that of the sequence in European patent (EP2634180A1), and more than 50% higher than that of the non-mutated aldo-keto reductase.

The whole cells of genetically engineered bacteria of aldo-keto reductase used in the Examples of the present invention were all prepared by this method.

The glucose dehydrogenases used in the Examples and Comparative Examples of the present invention were all commercial enzymes purchased from sigma-aldrich.

The algorithm for the ee value of enantiomer overexpression was:

$$ee(syn)=([R,R]-[S,S])/([R,R]+[S,S])$$

$$ee(anti)=([R,S]-[S,R])/([R,S]+[S,R])$$

$$de=\{([R,S]+[S,R])-([R,R]+[S,S])\}/\{([R,S]+[S,R])+([R,R]+[S,S])\}.$$

Enzymatic reduction reaction, with the reaction formula as follows:

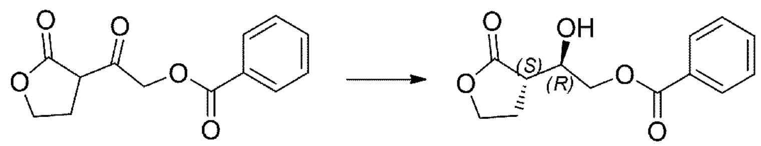

Step 1: The reaction was carried out in a 1 L shake flask, and the reaction system was controlled at the volume of 300 mL. In the shake flask, the broken enzyme solution of the whole cells of the genetically engineered bacteria of

aldo-keto reductase was suspended in 260 mL of sterilized potassium phosphate buffer solution. Glucose dehydrogenase was added to the shake flask, the cells were broken by ultrasound for 50min, then 25 g glucose, 0.42 g NADP+ were added to the shake flask. Then 8 g of the reactant was weighed and dissolved in 40 mL of DMSO, the DMSO solution in which the substrate dissolved was slowly poured into the shake flask. After 2 hours of reaction, 12 g of glucose was further added, the amount of the whole cells of the genetically engineered bacteria of aldo-keto reductase fed was 75 g/L, the amount of glucose dehydrogenase fed was 25 mg/L, and the temperature of the conversion system was controlled at 37°C. The conversion reaction was carried out in a shaker, the rotation speed of the shaker was controlled at 200 r/min, the conversion time was 12 h, and the conversion liquid of the target product was obtained, with a conversion rate of 97.8%.

Step 2: The conversion solution of the target product obtained in step 1 was purified, an equal volume of ethyl acetate was added to the reaction system, the extraction was carried out at 37°C for 15 min, repeated for 3 times, the ethyl acetate layer was collected by centrifugation, and 5% anhydrous magnesium sulfate was added to the collected ethyl acetate layer and shaken for 15 min, after that, the magnesium sulfate was removed by filtration. Then the ethyl acetate layer after the removal of water was concentrated under reduced pressure at elevated temperature, 7.41g of the target product was obtained, with a de value of 96.2% and an ee (anti) value of 99.5%.

Example 4:

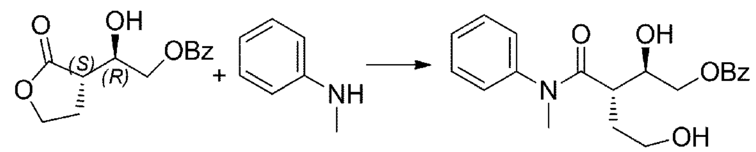

Cp1 Cp2

DCM (80ml) and $AlCl_3$ (5.6 g) were added to a 250 mL four-necked flask successively, N-methylaniline (17.1 g) was added dropwise, after dropwise addition, then the solution of the compound of formula Cp1 (8.0 g Cp1 dissolved in 80 mL of DCM) was added dropwise. After the dropwise addition, the temperature was kept. The reaction solution was dropped into dilute sulfuric acid (10.1 g sulfuric acid dissolved in 200 mL water) and stirred while dropping. After the dropwise addition, the pH was measured. The reaction mixture was stirred and separated into layers, then liquid separation was performed. The acid water layer was extracted with 100 mL DCM. The mixture was separated into layers, the organic layers were combined, and washed with 5% $NaHCO_3$ solution. The liquid separation was performed, and the organic layer was washed with 100 mL of water. The liquid separation was performed, and the organic layer was dried with $Na_2SO_4$. The organic layer was subjected to suction filtration, the filtrate was concentrated under reduced pressure at 50°C to obtain a yellow oily substance, and 27 mL EA and 27 mL heptane were added and stirred at 60°C until dissolved to clear. The temperature was slowly lowered to 0°C to crystallize. The obtained crystal was subjected to suction filtration, and rinsed with 20 mL of mixture (EA:heptane=1:1), and dried to obtain 10 g of white-like solid, compound of formula Cp2 (purity>98%).

Example 5:

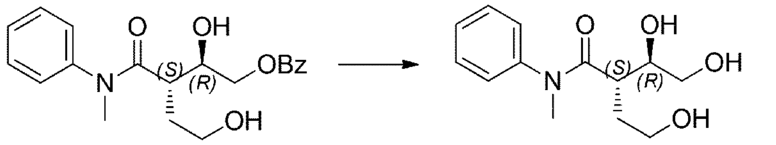

Cp2 Cp3

The compound of formula Cp2 (3.57 g) and methanol (15.00 mL) were added to a reaction flask successively, and 0.15 g caustic soda liquid was added dropwise under stirring, after the temperature was kept at -20~30°C, 5% sulfuric acid was added dropwise to adjust the pH, 0.1 g sodium bicarbonate was added, the mixture was concentrated under reduced pressure in a water bath at 30-35°C to remove methanol. After decompression, water was added, then DCM was added for extraction, the organic phases were combined, and concentrated under reduced pressure at 40°C

to obtain 2.50 g of product with a yield of about 99%.

Example 6:

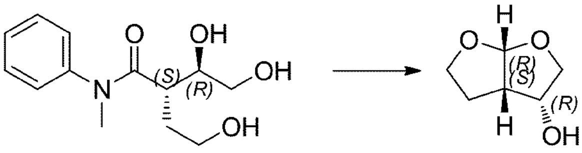

Cp3

The compound of formula Cp3 (2.50 g) and THF (20.00 mL) were added to a reaction flask successively, the inner temperature was cooled down, $LiAlH_4$ (1.50 g) was slowly added in batches, after addition the mixture was slowly warmed to room temperature (20-25°C) and stirred with the temperature kept. After the reaction, the inner temperature was cooled down, 10% dilute hydrochloric acid was slowly added dropwise to adjust the pH, and after the addition, the solution was slowly warmed to room temperature (20-25°C) and stirred. The inner temperature was cooled down, and 4% NaOH aqueous solution was slowly added to adjust the pH of the reaction solution. After adjusting the pH, toluene was added and stirred for extraction, the toluene layers were combined, the water layer was taken, DCM was added to the water layer under stirring for extraction, and the DCM layers were combined, the combined DCM layer was taken and an appropriate amount of anhydrous sodium sulfate was added to the DCM layer to dry. 1.0 g of colorless oily substance was obtained after concentration under reduced pressure at 40°C to dryness, with a yield of 80.0%.

Example 7:

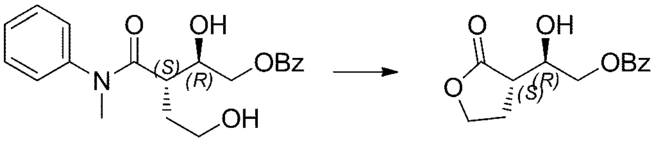

Cp2 Cp4

The compound of formula Cp2 (3.57 g), THF (25.00 mL), tetramethyldisiloxane (1.34 g), and titanium tetraisopropoxide (2.84 g) were added to a reaction flask successively, and stirred at 20-30°C for 6-24h, then EA (50 mL) was added, and 5% sulfuric acid was added dropwise to adjust the pH, the mixture was allowed to stand to separate into layers. The water layer was further extracted once with EA (20 mL), the organic layers were combined and 5% sodium bicarbonate was added thereto, and the EA was removed by concentration under reduced pressure in a water bath at 30-45°C. After decompression, EA (5 mL) was added for making slurry, then the solution was filtrated and dried to obtain 2.20 g of white or white-like solid compound of formula Cp4, with a yield of about 90%.

Example 8:

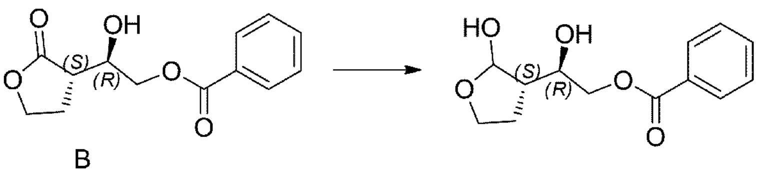

B

The solid compound of formula B ($R_1$ is benzoyl) (20.00 g) was added to a 500 mL dry and clean four-necked flask, and toluene was added and stirred, vacuum replacement was performed under nitrogen protection, the mixture

was cooled down under nitrogen protection, and toluene was added to a constant-pressure funnel under the protection of nitrogen, and 70% Red-Al solution (26.50 g, 26.00 mL) was added to the constant-pressure funnel under the protection of nitrogen. When the preparation was completed, after the reaction solution was cooled to -15~-10°C, the Red-Al was added, the temperature was controlled, after the dropwise addition, the temperature was kept. Pure water and ethyl acetate were added to a 1000 mL clean four-necked flask successively, sulfuric acid was added to the 1000 mL clean four-necked flask under stirring, then the solution was cooled down, after the temperature was kept constant, the reaction solution was dropped into the sulfuric acid solution, the temperature of the reaction liquid was controlled, the sulfuric acid solution was controlled at a temperature of 0-10°C, after the dropwise addition, ethyl acetate was added and then stirred; 10% sodium bicarbonate aqueous solution was added to a clean 1000 mL four-necked flask, the solution was cooled down, stop stirring, and the reaction solution was allowed to stand to separate into layers, the upper organic layer was transferred into the 10% sodium bicarbonate aqueous solution and stirred; the lower acid water layer was extracted by adding ethyl acetate, then allowed to stand to separate into layers, and the acid water layer was discarded, and the second ethyl acetate layer was transferred to 10% sodium bicarbonate aqueous solution and stirred, the temperature was controlled, and the solution was allowed to stand to separate into layers, ethyl acetate was added to the alkaline water layer for extraction and stirred, the temperature was controlled, pure water was added to the alkali-washed organic layer for washing, stirred, the temperature was controlled, the solution was allowed to separate into layers, the water-washed organic layer was obtained for further use, and the water-washed water layer was further extracted with ethyl acetate, and allowed to stand to separate into layers, and the water layer was discarded, the organic layers were combined, dried over sodium sulfate, subjected to suction filtration, and rinsed with ethyl acetate. The filtrate was concentrated under reduced pressure at 40-70°C to obtain about 17 g of an oily substance with a yield of 84.33%.

Example 9:

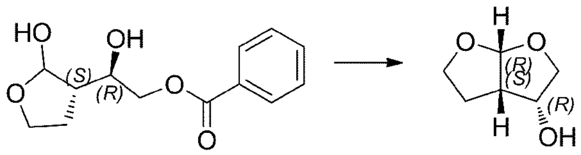

C

Methanol (10.00 mL) and the compound of formula C (R1 is benzoyl) (1.00 g) were added to a reaction flask successively, cooled to -15~-5°C, sodium hydroxide aqueous solution (10.00 mL) was added dropwise, and after the dropwise addition, the solution was stirred with the temperature kept until the raw material has been reacted completely. 10% sulfuric acid aqueous solution (4.00 g) was added dropwise, after the dropwise addition, the solution was continued reacting with the temperature kept, after the temperature start to change, saturated sodium carbonate aqueous solution was added to adjust the pH. After the methanol was removed by distillation under reduced pressure at elevated temperature, methyl tert-butyl ether was added for extraction, the organic layer was separated, sodium chloride was added to the water layer until saturation and dichloromethane was further added for extraction, the water layer was separated for recovery, and the organic layer was distilled under reduced pressure to remove dichloromethane, 0.38 g of product was obtained with a yield of 73.77%.

Example 10:

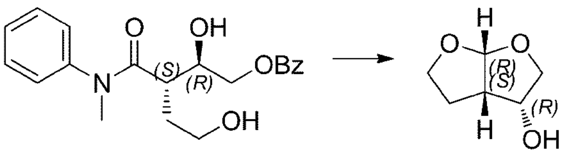

Cp2

The compound of formula Cp2 (3.50 g) and THF (20.00 mL) were added to a reaction flask successively, and the inner temperature was cooled down, $LiAlH_4$ (1.50 g) was slowly added in batches, after addition the mixture was

slowly warmed to room temperature (20-25°C) and stirred with the temperature kept. After the reaction was completed, the inner temperature was cooled down, 10% dilute hydrochloric acid was slowly added dropwise to adjust the pH, and after the addition, the solution was slowly warmed to room temperature (20-25°C) and stirred. The inner temperature was cooled down, and 4% NaOH aqueous solution was slowly added to adjust the pH of the reaction solution. After adjusting the pH, toluene was added and stirred for extraction, the toluene layers were combined, the water layer was taken, DCM was added to the water layer under stirring for extraction, and DCM layers were combined, the combined DCM layer was taken and an appropriate amount of anhydrous sodium sulfate was added to the DCM layer to dry. 1.0 g of colorless oily substance was obtained after concentration under reduced pressure at 40°C to dryness, with a yield of 80.0%.

Example 11:

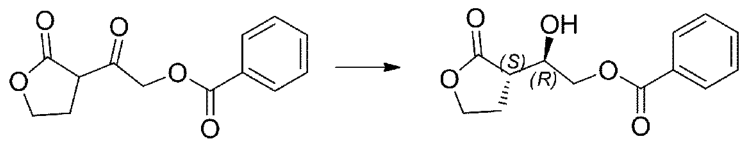

The preparation of the enzyme was the same as in Example 3.

Step 1: The reaction was carried out in a 5 L beaker, and the reaction system was controlled at the volume of 2 L. In the shake flask, the whole cells of the genetically engineered bacteria of aldo-keto reductase was suspended in 1.7 L of sterilized potassium phosphate buffer solution. Glucose dehydrogenase was added to the shake flask, the cells were broken by ultrasound for 50min, then 25 g glucose, 0.42 g NADP+ were added to the beaker. Then 80 g of the substrate was weighed and dissolved in 300 mL of DMSO, the DMSO solution in which the substrate dissolved was slowly poured into the shake flask. After 2 hours of reaction, 12 g of glucose was further added, the amount of the whole cells of the genetically engineered bacteria of aldo-keto reductase fed was 75 g/L, the amount of glucose dehydrogenase fed was 25 mg/L, and the temperature of the conversion system was controlled to 37°C. The conversion reaction was carried out in a beaker, the rotation speed of the magneton was controlled at 200 r/min, the conversion time was 12 h, and the conversion liquid of the target product was obtained with a conversion rate of 97.8%.

Step 2: The conversion solution containing the intermediate of Darunavir, the compound of formula VIII obtained in step 1, was purified, and the purification steps was as in Example 3, 77.10 g of the target compound was obtained. The prepared target compound had a de value of 95.3%, an ee (anti) value of 99.6%.

Example 12:

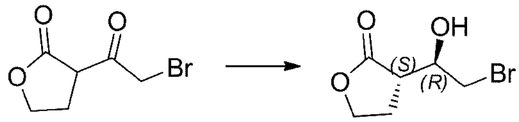

Step 1: The reaction was carried out in a 1 L shake flask, and the reaction system was controlled at the volume of 300 mL. In the shake flask, the whole cells of the genetically engineered bacteria of aldo-keto reductase was suspended in 250 mL of sterilized deionized water. Glucose dehydrogenase was added to the shake flask, and 10 mL of 2.5 mol/L glucose, 0.26 g NADP+ were added. Then 10 g of the substrate was weighed and dissolved in 30 mL of butyl acetate, the butyl acetate solution in which the substrate was dissolved was slowly poured into the shake flask. After 1 hour of reaction, 10 mL of 2.5mol/L glucose was further added, the amount of the whole cells of the genetically engineered bacteria of aldo-keto reductase fed was 75 g/L, the amount of glucose dehydrogenase was 25 mg/L, and the temperature of the conversion system was controlled at 37°C. The conversion reaction was carried out in a shaker, the rotation speed of the shaker was controlled at 200 r/min, the conversion time was 12 h, and the conversion liquid of the target product was obtained with a conversion rate of 97.8%. Step 2: The conversion solution of the target compound obtained in step 1 was purified, an equal volume of ethyl acetate was added to the reaction system, the extraction was carried out at 37°C for 15 min, repeated for 3 times, the ethyl acetate layer was collected by centrifugation, and 5% anhydrous magnesium sulfate was added to the collected ethyl acetate layer and shaken for 15 min, after that, the magnesium sulfate was removed by filtration. Then the ethyl acetate layer after the removal of water was concentrated under reduced

pressure at elevated temperature, 9.55 g of the target compound was obtained, the prepared target compound had a de value of 99.1%, an ee (anti) value of the enantiomer is 99.7%. $^1$H NMR (600 MHz, $CDCl_3$) $\delta$ 2.269~2.301(m, 1 H, $J$=6 Hz), 2.367~2.404(m, 1 H), 2.954~2.993(m, 1 H, $J$ = 6 Hz), 3.438~3.466(m, 1 H), 3.520~ 3.549(m, 1 H), 4.227~4.269(m, 1 H), 4.298~4.326(m, 1 H), 4.391~4.420(m, 1 H). MS(ESI):m/z 210.03 $[M+H]^+$.

Example 13:

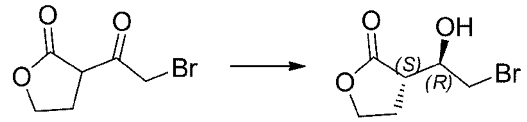

Step 1: The reaction was carried out in a 5 L beaker, and the reaction system was controlled at the volume of 2 L. In the beaker, the whole cells of the genetically engineered bacteria of aldo-keto reductase was suspended in 1.5 L of sterilized deionized water. Glucose dehydrogenase was added, and 100 mL of 2.5 mol/L glucose, 3 g NADP+ were added. Then 100 g of substrate was weighed and dissolved in 300 mL of butyl acetate, the butyl acetate solution in which the substrate was dissolved was slowly poured into the beaker. After 1 hour of reaction, 100 mL of 2.5 mol/L glucose was further added, the amount of the whole cells of the genetically engineered bacteria of aldo-keto reductase fed was 100 g/L, the amount of glucose dehydrogenase fed was 25 mg/L, and the temperature of the conversion system was controlled at 28°C. The conversion reaction was carried out in a beaker, the rotation speed of the magneton was controlled at 200 r/min, the conversion time was 12 h, and the conversion liquid of the target was obtained with a conversion rate of 97.8%.

Step 2: The conversion solution of the target obtained in step 1 was purified, and the purification steps was as in Example 3, 9.42 g of the target was obtained. The the prepared target had a de value of 96.9%, an ee (anti) value of the enantiomer is 99.4%.

Example 14:

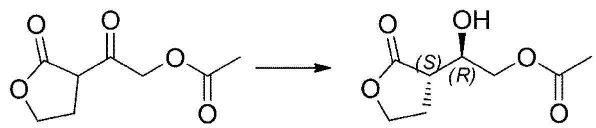

Step 1: The reaction was carried out in a 1 L shake flask, and the reaction system was controlled at the volume of 300 mL. In the shake flask, the whole cells of the genetically engineered bacteria of aldo-keto reductase was suspended in 250 mL of sterilized deionized water. Glucose dehydrogenase was added, and 10 mL of 2.5 mol/L glucose, 0.26 g NADP+ were added. Then 10 g of substrate was weighed and dissolved in 30 mL of butyl acetate, the butyl acetate solution in which the substrate was dissolved was slowly poured into the shake flask. After 1 hour of reaction, 10 mL of 2.5 mol/L glucose was further added, the amount of the whole cells of the genetically engineered bacteria of aldo-keto reductase fed was 75 g/L, the amount of glucose dehydrogenase fed was 25 mg/L, and the temperature of the conversion system was controlled at 37°C. The conversion reaction was carried out in a shaker, the rotation speed of the shaker was controlled at 200 r/min, the conversion time was 12 h, and the conversion liquid of the target was obtained with a conversion rate of 97.8%.

Step 2: The conversion solution of the target compound obtained in step 1 was purified, and the purification steps was as in Example 3, 9.37 g of the target was obtained. The prepared target compound had a de value of 97.1%, an ee (anti) value of 99.5%.

Example 15:

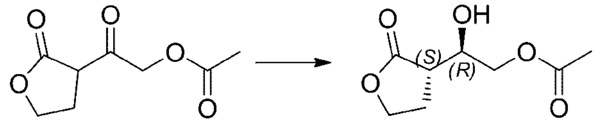

Step 1: The reaction was carried out in a 5 L beaker, and the reaction system was controlled at the volume of 2 L. In the beaker, the whole cells of the genetically engineered bacteria of aldo-keto reductase was suspended in 1.6 L of sterilized deionized water. Glucose dehydrogenase was added, and 100 mL of 2.5 mol/L glucose, 2.5 g NADP+ were added. Then 100 g of substrate was weighed and dissolved in 200 mL of butyl acetate, the butyl acetate solution in which the substrate was dissolved was slowly poured into the beaker. After 1 hour of reaction, 100 mL of 2.5 mol/L glucose was further added, the amount of the whole cells of the genetically engineered bacteria of aldo-keto reductase fed was 50 g/L, the amount of glucose dehydrogenase fed was 25 mg/L, and the temperature of the conversion system was controlled at 25°C. The conversion reaction was carried out in a beaker, the rotation speed of the magneton was controlled at 200 r/min, the conversion time was 12h, and the conversion liquid of the target compound was obtained with a conversion rate of 97.8%.

Step 2: The conversion solution of the target compound obtained in step 1 was purified, and the purification step was as in Example 3, 93.1 g of the target compound was obtained. The prepared target compound had a de value of 95.6%, and an ee (anti) value of 99.6%.

Example 16: Comparative Example

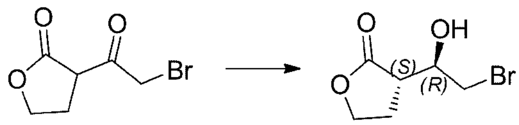

Step 1: The reaction is carried out in a 1 L shake flask, and the reaction system was controlled at the volume of 300 mL. In the shake flask, the whole cells of the genetically engineered bacteria of aldo-keto reductase was suspended in 250 mL of sterilized deionized water. The coding sequence of the aldo-keto reductase gene of the whole cell of the genetically engineered bacteria of the aldo-keto reductase used was as the sequence published in the European patent EP2634180 (ie SEQ ID NO 12 shown in the patent EP2634180), and the sequence was synthesized by whole gene (synthesized by Genscript Biotechnology Co., Ltd.), the preparation method was as in Example 3. The glucose dehydrogenase was added, and 10 mL of 2.5 mol/L glucose, 0.26 g NADP+ were added. Then 10 g of substrate was weighed and dissolved in 30 mL of butyl acetate, the butyl acetate solution in which the substrate was dissolved was slowly poured into the beaker. After 1 hour of reaction, 10 mL of 2.5 mol/L glucose was further added, the amount of the whole cells of the genetically engineered bacteria of aldo-keto reductase fed was 75 g/L, the amount of glucose dehydrogenase fed was 25 mg/L, and the temperature of the conversion system was controlled at 37°C. The conversion reaction was carried out in a shaker, the rotation speed of the shaker was controlled at 200 r/min, the conversion time was 12 h.

The purification step was as in Example 3, 8.11 g of the target compound was obtained. The prepared target compound had a de value of 85.1%, and an ee (anti) value of the enantiomer of 93.3%.

Example 17: Comparative Example

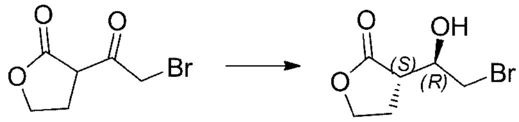

Step 1: The reaction is carried out in a 1 L shake flask, and the reaction system was controlled at the volume of 300 mL. In the shake flask, the whole cells of the genetically engineered bacteria of aldo-keto reductase of Saccharomyces kudriavzevii was suspended in 250 mL of sterilized deionized water. The coding sequence of the aldo-keto reductase gene of the whole cell of the genetically engineered bacteria of the aldo-keto reductase used was shown in SEQ ID NO 3 (the coding sequence of aldo-keto reductase gene has not been artificially designed), and the sequence was synthesized by whole gene (synthesized by Genscript Biotechnology Co., Ltd.), the preparation method was as in

Example 3. The glucose dehydrogenase was added, and 10 mL of 2.5 mol/L glucose, 0.26 g NADP+ were added. Then 10 g of substrate was weighed and dissolved in 30 mL of butyl acetate, the butyl acetate solution in which the substrate was dissolved was slowly poured into the beaker. After 1 hour of reaction, 10 mL of 2.5 mol/L glucose was further added, the amount of the whole cells of the genetically engineered bacteria of aldo-keto reductase fed was 75 g/L, the amount of glucose dehydrogenase fed was 25 mg/L, and the temperature of the conversion system was controlled at 37°C. The conversion reaction was carried out in a shaker, the rotation speed of the shaker was controlled at 200 r/min, the conversion time was 12 h.

The purification step was as in Example 3, 7.73 g of the target compound was obtained. The prepared target compound had a de value of 79.6%, and a ee (anti) value of the enantiomer of 88.7.

Serial number of SEQ ID NO 1:

Met Ser Asp Leu Phe Lys Pro Ala Pro Glu Pro Pro Thr Glu Leu Gly

Arg Leu Arg Val Leu Ser Lys Thr Ala Gly Ile Arg Val Ser Pro Leu

Ile Leu Gly Gly Ala Ser Ile Gly Asp Ala Trp Ser Gly Phe Met Gly

Ser Met Asn Lys Glu Gln Ala Phe Glu Leu Leu Asp Ala Phe Tyr Glu

Ala Gly Gly Asn Cys Val Asp Thr Ala Asn Ser Tyr Gln Asn Glu Glu

Ser Glu Ile Trp Ile Gly Glu Trp Met Lys Ser Arg Lys Leu Arg Asp

Gln Ile Val Ile Ala Thr Lys Phe Thr Gly Asp Tyr Lys Lys Tyr Glu

Val Gly Gly Gly Lys Ser Ala Asn Tyr Cys Gly Asn His Lys His Ser

Leu His Val Ser Val Arg Asp Ser Leu Arg Lys Leu Gln Thr Asp Trp

Ile Asp Ile Leu Tyr Val His Trp Trp Asp Tyr Met Ser Ser Ile Glu

Glu Val Met Asp Ser Leu His Ile Leu Ile Gln Gln Gly Lys Val Leu

Tyr Leu Gly Val Ser Asp Thr Pro Ala Trp Val Val Ser Ala Ala Asn

Asn Tyr Ala Thr Ser His Gly Lys Thr Pro Phe Ser Ile Tyr Gln Gly
Lys Trp Asn Val Leu Asn Arg Asp Phe Glu Arg Asp Ile Ile Pro Met
Ala Arg His Phe Gly Met Ala Leu Ala Pro Trp Asp Val Met Gly Gly
Gly Lys Phe Gln Ser Lys Lys Ala Met Glu Glu Trp Lys Lys Asn Gly
Glu Gly Leu Arg Thr Ala Val Gly Gly Pro Glu Gln Thr Glu Leu Glu
Val Lys Ile Ser Glu Ala Leu Asn Lys Ile Ala Glu Glu His Gly Thr
Glu Ser Val Thr Ala Ile Ala Ile Ala Tyr Val Arg Ser Lys Ala Lys
Asn Val Phe Pro Leu Val Gly Gly Arg Lys Ile Glu His Leu Lys Gln
Asn Ile Glu Ala Leu Ser Ile Lys Leu Thr Pro Glu Gln Ile Glu Tyr
Leu Glu Ser Ile Val Thr Phe Asp Val Gly Phe Pro Lys Ser Asn Ile
Gly Asp Asp Pro Ala Val Thr Lys Lys Leu Ser Pro Leu Thr Ser Met
Ser Ala Arg Ile Ser Phe Asp Asn

Serial number of SEQ ID NO 2:

atgagcgatc tgtttaaacc ggcgccggaa ccgccgaccg aactgggccg cctgcgcgtg 60
ctgagcaaaa ccgcgggcat tcgcgtgagc ccgctgattc tgggcggcgc gagcattggc 120
gatgcgtgga gcggctttat gggcagcatg aacaaagaac aggcgtttga actgctggat 180
gcgttttatg aagcgggcgg caactgcgtg gataccgcga acagctatca gaacgaagaa 240
agcgaaattt ggattggcga atggatgaaa agccgcaaac tgcgcgatca gattgtgatt 300
gcgaccaaat ttaccggcga ttataaaaaa tatgaagtgg gcggcggcaa aagcgcgaac 360
tattgcggca accataaaca tagcctgcat gtgagcgtgc gcgatagcct gcgcaaactg 420
cagaccgatt ggattgatat tctgtatgtg cattggtggg attatatgag cagcattgaa 480
gaagtgatgg atagcctgca tattctgatt cagcagggca aagtgctgta tctgggcgtg 540
agcgataccc cggcgtgggt ggtgagcgcg gcgaacaact atgcgaccag ccatggcaaa 600
accccgttta gcatttatca gggcaaatgg aacgtgctga accgcgattt tgaacgcgat 660
attattccga tggcgcgcca ttttggcatg gcgctggcgc cgtgggatgt gatgggcggc 720
ggcaaatttc agagcaaaaa agcgatggaa gaatggaaaa aaaacggcga aggcctgcgc 780
accgcggtgg gcggcccgga acagaccgaa ctggaagtga aaattagcga agcgctgaac 840

aaaattgcgg aagaacatgg caccgaaagc gtgaccgcga ttgcgattgc gtatgtgcgc 900
agcaaagcga aaaacgtgtt tccgctggtg ggcggccgca aaattgaaca tctgaaacag 960
aacattgaag cgctgagcat taaactgacc ccggaacaga ttgaatatct ggaaagcatt 1020
gtgacctttg atgtgggctt tccgaaaagc aacattggcg atgatccggc ggtgaccaaa 1080
aaactgagcc cgctgaccag catgagcgcg cgcattagct ttgataacta a 1131

Serial number of SEQ ID NO 3:

atgtctgatg tatttggacc tgcacctgaa ccacctaccg agttaggacg tctaagagtt 60
ctctctaaaa cagctggtat aagagtctct ccgctaatat tgggaggtat gtcgattggt 120
gacgcctggt caggattcat ggggtcaatg aacaaggagc gggcttttga gctgcttgat 180
gcctttttcg aggcaggtgg aaacttcatt gatactgcaa ataattacca aaatgaacag 240
tcagaggcat ggataggtga atggatggtt tcaagaaaat tgcgtgacca aattgttatt 300
gccaccaaat tcaccacaga ctataagaag tatgaagtgg gcaagggcag aagtgccaac 360
ttctgtggta atcacaagca tagtttacac gtaagtgtga gagattctct tcgcaaattg 420
cagactgatt ggattgacat tctctatgtt cactggtggg attatatgag ttcgatcgag 480
gaagttatgg atagtctgca tattcttgtg cagcagggca aggtcctcta cctgggagta 540
tctgatacac ctgcatgggt cgtgtctgct gcaaattact acgctacctc tcacgggaaa 600
actcccttca gcatctatca aggtaaatgg aatctgttga atagggactt tgagcgtgaa 660
attattccaa tggctaggca ttttggtatg gctctcgctc catgggatgt catgggaggg 720
ggaagatttc agagcaaaaa agctttagaa gaacggaaga agagtggaga gggcctgcgt 780
agctttgttg gtacatctga acagacggat gcagaggtta agatcagcga ggcattgtcg 840
aaggttgctg aggaacatgg cattgagtct gtcacagcta ttgccattgc ctatgtccgc 900
tccaaagcga agcatgtttt cccattggtt ggaggaagga aaattgagca cctcaaacaa 960
aatattgagg cgttgagtat taaattgaca ccaggacaga tagaatatct agaaagcatt 1020
gtcccattcg atgttgggtt ccctagcaat ttcatcggag atgatcctgc agttactaag 1080
aaacttgcat tccttccagc aatgtctgcc aagattgctt ttgacgatta g 1131

## Claims

1. An intermediate of (3R, 3aS, 6aR)-hexahydrofuro[2,3-b]-3-ol, compound of formula C or Cf, wherein having a structural formula as follows:

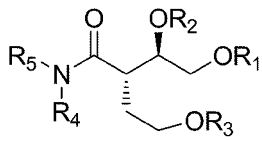

C ,

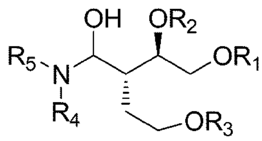

Cf ,

wherein, $R_1$, $R_2$, $R_3$ are hydrogen or hydroxy protecting groups; $R_4$, $R_5$ are the same or different and are phenyl, alkyl or substituted phenyl.

2. The compound according to claim 1, wherein the hydroxy protecting group is alkyl, silyl, $C_{2-11}$ acyl, $C_{4-9}$ cycloalkenyl, aryl, aralkyl, aroyl, phenyl, substituted phenyl; the silyl is tetramethylsilyl, trimethylsilyl, triethylsilyl, tri-n-butylsilyl, tert-butyldimethylsilyl; the alkyl is a $C_1$-$C_8$ alkyl; the aryl group is phenyl, furanyl, thienyl or indolyl; the substituted phenyl is alkyl-substituted phenyl, alkoxyalkyl-substituted phenyl, nitroalkyl-substituted phenyl or halogen-substituted phenyl; the alkyl-substituted phenyl is benzyl, benzhydryl, trityl; the alkoxyalkyl-substituted phenyl is p-methoxybenzyl; the nitroalkyl-substituted phenyl is p-nitrobenzyl; the halogen-substituted phenyl is p-chlorophenyl.

3. A method for preparing an intermediate of (3R, 3aS, 6aR)-hexahydrofuro[2,3-b]-3-ol, compound of formula $C_L$, wherein the intermediate is prepared by reacting a compound of formula Cp with an amine compound,

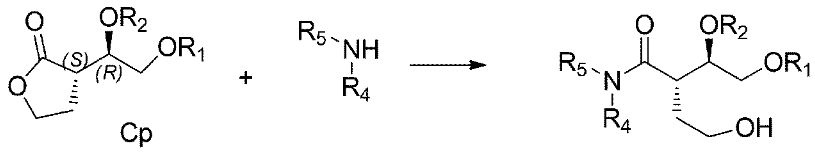

$C_L$ ,

wherein, the definitions of $R_1$, $R_2$, $R_4$, $R_5$ are the same as in claim 1.

4. A method for preparing an intermediate of (3R, 3aS, 6aR)-hexahydrofuro[2,3-b]-3-ol, compound of formula C-1, wherein the intermediate is prepared by reacting a compound of formula Cp with an N-methylaniline compound,

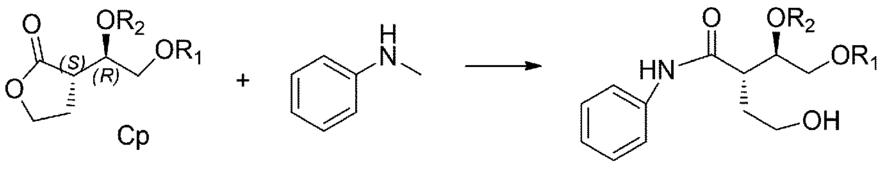

C-1

wherein, the definitions of $R_1$, $R_2$ are the same as in claim 1.

**5.** A method for preparing an intermediate of (3R, 3aS, 6aR)-hexahydrofuro[2,3-b]-3-ol, wherein the intermediate is prepared by enzymatic reduction reaction to construct chirality,

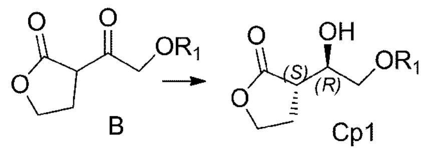

,

wherein, $R_1$ is a hydrogen or hydroxyl protection group;
the enzyme is an aldo-keto reductase having an amino acid sequence that is the protein shown in SEQ ID NO: 1, or the protein having aldo-keto reductase activity obtained from SEQ ID NO: 1 after substitution, deletion or addition of one or more amino acid residues, or the protein having 80% homology with the amino acid sequence shown in SEQ ID NO:1 and having aldo-keto reductase activity.

**6.** A method for preparing an intermediate compound of (3R, 3aS, 6aR)-hexahydrofuro[2,3-b]-3-ol, wherein the intermediate is prepared by enzymatic reduction reaction to construct chirality, and further by introducing a protecting group,

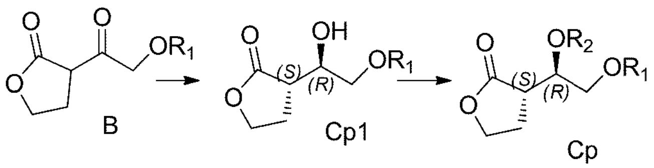

,

wherein, the definition of $R_1$ is the same as in claim 1, $R_2$ is a hydroxy protecting group, and the enzyme is the same as in claim 5.

**7.** The preparation method according to claim 5 or 6, wherein the compound of formula B is prepared from the compound of formula A2 by acylation reaction, and the reaction formula is as follows:

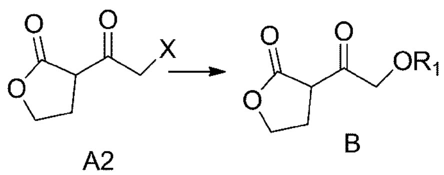

,

wherein, X is halogen, and the definition of $R_1$ is the same as in claim 1.

**8.** The preparation method according to claim 7, wherein the compound of formula A2 is prepared from the compound of formula A1 by halogenation reaction, and the reaction formula is as follows:

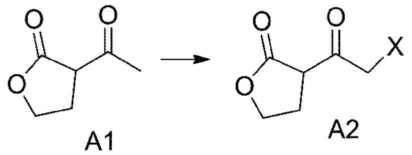

A1 A2

,

wherein, X is halogen.

**9.** A preparation method of an intermediate of (3R, 3aS, 6aR)-hexahydrofuro[2,3-b]-3-ol, compound of formula C-i, wherein the intermediate is prepared from the compound of formula C-1 by deprotection reaction,

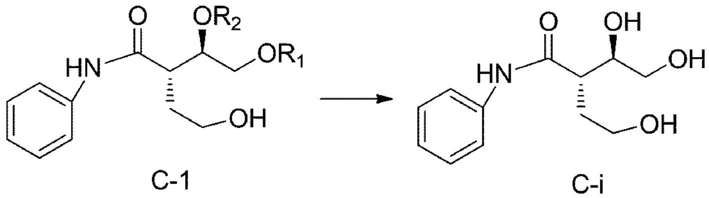

C-1 C-i

,

wherein, the definitions of $R_1$, $R_2$ are the same as in claim 1.

**10.** A preparation method of an intermediate of (3R, 3aS, 6aR)-hexahydrofuro[2,3-b]-3-ol, compound of formula Cf, wherein the intermediate is prepared from the compound of formula $C_L$ by reduction reaction,

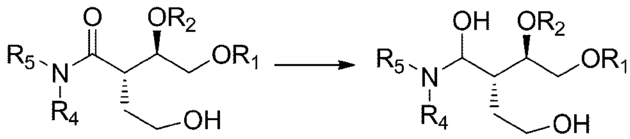

$C_L$ Cf

,

wherein, the definitions of $R_1$, $R_2$, $R_4$, $R_5$ are the same as in claim 1.

**11.** A preparation method of an intermediate of (3R, 3aS, 6aR)-hexahydrofuro[2,3-b]-3-ol, compound of formula Cf-1, wherein the intermediate is prepared from the compound of formula C-i by reduction reaction,

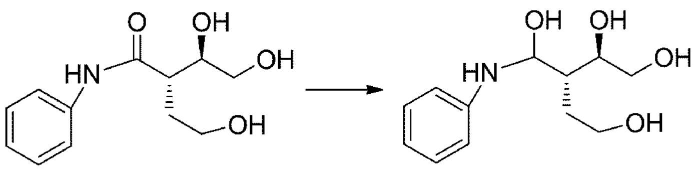

C-i Cf-1

.

**12.** A preparation method of (3R, 3aS, 6aR)-hexahydrofuro[2,3-b]-3-ol, wherein (3R, 3aS, 6aR)-hexahydrofuro[2,3-b]-3-ol is prepared from the compound of formula Cf by ring closure reaction,

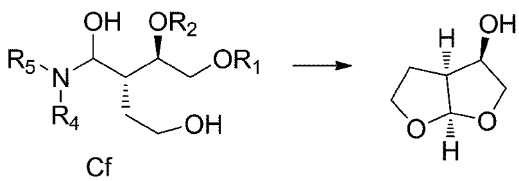

Cf

,

wherein, the definitions of $R_1$, $R_2$, $R_4$, $R_5$ are the same as in claim 1.

**13.** A preparation method of (3R, 3aS, 6aR)-hexahydrofuro[2,3-b]-3-ol, wherein (3R, 3aS, 6aR)-hexahydrofuro[2,3-b]-3-ol is prepared from the compound of formula Cf-1 by ring closure reaction,

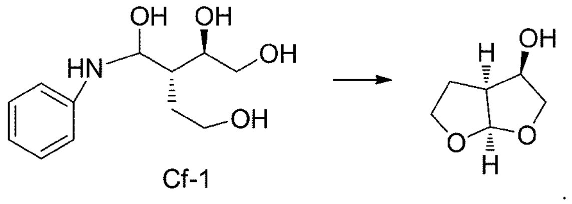

Cf-1

.

**14.** A preparation method of (3R, 3aS, 6aR)-hexahydrofuro[2,3-b]-3-ol, wherein (3R, 3aS, 6aR)-hexahydrofuro[2,3-b]-3-ol is prepared by halogenation reaction, acylation reaction, enzymatic reduction reaction, reaction with amine compounds, reduction ring-closure reaction,

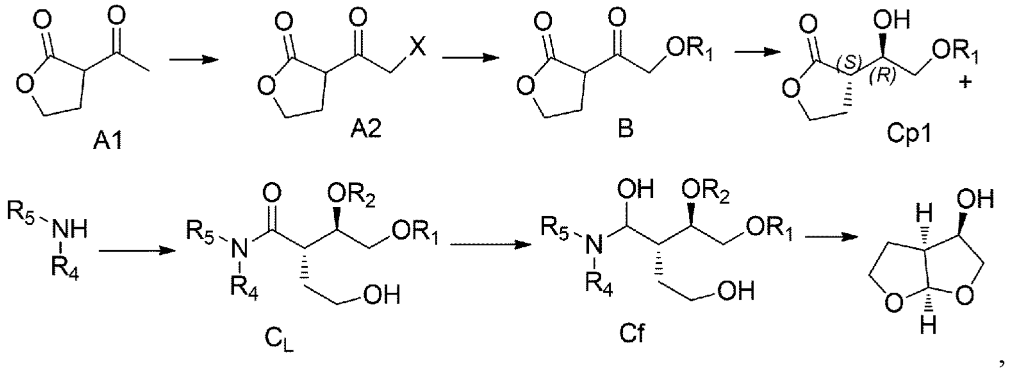

A1 A2 B Cp1

$C_L$ Cf

,

wherein, X is halogen, and the definitions of $R_1$, $R_2$, $R_4$, $R_5$ are the same as in claim 1.

**15.** A preparation method of (3R, 3aS, 6aR)-hexahydrofuro[2,3-b]-3-ol, wherein (3R, 3aS, 6aR)-hexahydrofuro[2,3-b]-3-ol is prepared by halogenation reaction, acylation reaction, enzymatic reduction reaction, reaction with amine compounds, deprotection reaction, reduction ring-closure reaction,

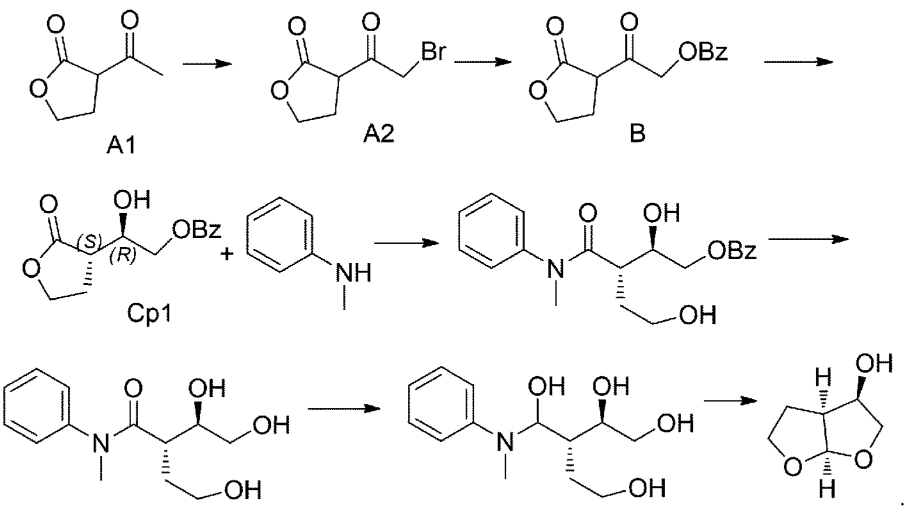

A1 A2 B

Cp1

.

**16.** The preparation method according to claim 5, wherein the nucleotide sequence of the aldo-keto reductase gene is SEQ ID NO: 2.

**17.** The preparation method according to claim 5, wherein the aldo-keto reductase is whole cell of genetically engineered bacteria, broken enzyme solution, freeze-dried powder, or immobilized enzyme or immobilized cells.

**18.** The preparation method according to claim 5, wherein the amount of the whole cell of the genetically engineered bacteria of aldo-keto reductase fed in the reaction system is at the range of 10-100 g/L, and the conversion temperature is 25-37°C.

**19.** The preparation method according to claim 5, wherein the reaction is carried out in the presence of a solvent.

**20.** The preparation method according to claim 19, wherein the solvent is a mixed solvent that is composed of water or a buffer solution and an organic solvent.

**21.** The preparation method according to claim 20, wherein the buffer solution is selected from one or more of phosphate buffer solution, carbonate buffer solution, Tri-HCl buffer solution, citrate buffer solution or MOPS buffer solution.

**22.** The preparation method according to claim 20, wherein the organic solvent is selected from one or more of DMSO, ethyl acetate, butyl acetate, isopropanol, DMF, TBME, dichloromethane, and vinyl acetate.

**23.** A preparation method of an intermediate of (3R, 3aS, 6aR)-hexahydrofuro[2,3-b]-3-ol, wherein the intermediate is prepared from the compound of formula $C_L$ by ring closure reaction,

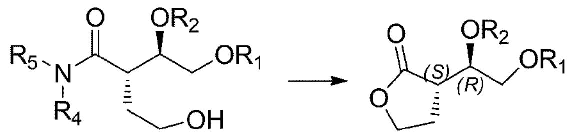

$C_L$ Cp ,

wherein, the definitions of $R_1$, $R_2$, $R_4$, $R_5$ are the same as in claim 1.

## INTERNATIONAL SEARCH REPORT

International application No.
**PCT/CN2018/097739**

**A. CLASSIFICATION OF SUBJECT MATTER**

C07C 213/06(2006.01)i; C07C 215/06(2006.01)i; C07C 215/10(2006.01)i; C07C 215/12(2006.01)i; C07C 215/16(2006.01)i; C07C 217/08(2006.01)i; C07C 217/76(2006.01)i

According to International Patent Classification (IPC) or to both national classification and IPC

**B. FIELDS SEARCHED**

Minimum documentation searched (classification system followed by classification symbols)

C07C213/-; C07C215/; C07C217/-

Documentation searched other than minimum documentation to the extent that such documents are included in the fields searched

Electronic data base consulted during the international search (name of data base and, where practicable, search terms used)

WPI, EPODOC, CNPAT, CNKI, STN (REG, CAPLUS): 达芦那韦, 六氢呋喃, 呋喃醇, 中间体, 制备, darunavir, furan, synthesis

**C. DOCUMENTS CONSIDERED TO BE RELEVANT**

| Category* | Citation of document, with indication, where appropriate, of the relevant passages | Relevant to claim No. |
|---|---|---|
| X | WO 2010075887 A1 (OXYRANE UK LTD. ET AL.) 08 July 2010 (2010-07-08) entire document, particularly description, page 25, scheme 1 and page 29, paragraph 1, embodiment, step C | 1-2, 12-13 |
| Y | | 3-4, 14-15 |
| Y | EP 2634180 A1 (LONZA AG) 04 September 2013 (2013-09-04) entire document, particularly description, paragraphs [0008]-[0020] and [0066] | 14-15 |
| Y | MEKHAEL, M.K.G. et al. "Reactions with 4-Hydroxy-2-Methylbutananilides: Unexpected Formation of a Cyclopropanecarboxamide" *Helvetica Chimica Acta,* Vol. 94, No. (1), 31 December 2011 (2011-12-31), ISSN: 0018-019X, pp. 28-37, particularly p. 29, scheme 2 | 3-4, 14-15 |
| Y | LESIMPLE, P. et al. "Aluminum Chloride Mediated Aminolysis of Lactones: a General Method for the Preparation of ω-hydroxyalkylamides" *Synthesis,* No. no. 4, 31 December 1991 (1991-12-31), ISSN: 0039-7881, pages 306-308, particularly page 306, right-hand column | 3-4, 14-15 |

☐ Further documents are listed in the continuation of Box C.  ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **04 December 2018** | **04 January 2019** |

| Name and mailing address of the ISA/CN | Authorized officer |
|---|---|
| **State Intellectual Property Office of the P. R. China No. 6, Xitucheng Road, Jimenqiao Haidian District, Beijing 100088 China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2018/097739**

**Box No. III Observations where unity of invention is lacking (Continuation of item 3 of first sheet)**

This International Searching Authority found multiple inventions in this international application, as follows:

[1] There are three inventions:

[2] The first invention: claims 1-4 and 9-15 relate to a (3R, 3aS, 6aS)-hexahydrofuro[2, 3-b]furan-3-ol intermediate compound of formula C or Cf, preparation method of the two compounds and a method for preparing (3R, 3aS, 6aS)-hexahydrofuro[2, 3-b]furan-3-ol through these two compounds.

[3] The second invention: claims 5-8 and 16-22 relate to a method for preparing the compound of formula Cp1 by means of enzymatic reduction.

[4] The third invention: claim 23 relates to a method for preparing the compound of formula Cp by the compound of formula $C_L$ through a ring closing reaction.

[5] There is no same or corresponding technical feature between the first, second and third inventions, and the same or corresponding technical features between the second and third inventions, i.e. the compound of formula Cp1 and the compound of formula Cp, are known compounds. There is no same or corresponding special technical feature between the two methods, and the methods are different inventive concepts. Therefore, the above-mentioned three inventions do not fall within a single general inventive concept, and therefore do not satisfy the requirement of unity of invention.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **claims 1-4 and 9-15**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

**INTERNATIONAL SEARCH REPORT**
**Information on patent family members**

International application No.
**PCT/CN2018/097739**

| Patent document cited in search report | | | Publication date (day/month/year) | Patent family member(s) | Publication date (day/month/year) |
|---|---|---|---|---|---|
| WO | 2010075887 | A1 | 08 July 2010 | None | |
| EP | 2634180 | A1 | 04 September 2013 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 201810325842
- CN 02817639
- CN 200580010400X
- CN 2003080109926
- EP 2634180 A1
- EP 2634180 A

SEQUENCE LISTING

<110> JIANGSU RUIKE MEDICAL SCIENCE AND TECHNOLOGY CO.,LTD

<120> Method for preparing hexahydrofuro-furanol derivative, Intermediate thereof and preparation method thereof

<130> ESP1V201528ZX

<160> 3

<170> PatentIn version 3.5

<210> 1
<211> 376
<212> PRT
<213> artifical sequence

<400> 1

```
Met Ser Asp Leu Phe Lys Pro Ala Pro Glu Pro Pro Thr Glu Leu Gly
1               5                   10                  15

Arg Leu Arg Val Leu Ser Lys Thr Ala Gly Ile Arg Val Ser Pro Leu
            20                  25                  30

Ile Leu Gly Gly Ala Ser Ile Gly Asp Ala Trp Ser Gly Phe Met Gly
        35                  40                  45

Ser Met Asn Lys Glu Gln Ala Phe Glu Leu Leu Asp Ala Phe Tyr Glu
    50                  55                  60

Ala Gly Gly Asn Cys Val Asp Thr Ala Asn Ser Tyr Gln Asn Glu Glu
65                  70                  75                  80

Ser Glu Ile Trp Ile Gly Glu Trp Met Lys Ser Arg Lys Leu Arg Asp
                85                  90                  95

Gln Ile Val Ile Ala Thr Lys Phe Thr Gly Asp Tyr Lys Lys Tyr Glu
            100                 105                 110

Val Gly Gly Gly Lys Ser Ala Asn Tyr Cys Gly Asn His Lys His Ser
        115                 120                 125

Leu His Val Ser Val Arg Asp Ser Leu Arg Lys Leu Gln Thr Asp Trp
    130                 135                 140

Ile Asp Ile Leu Tyr Val His Trp Trp Asp Tyr Met Ser Ser Ile Glu
145                 150                 155                 160

Glu Val Met Asp Ser Leu His Ile Leu Ile Gln Gln Gly Lys Val Leu
                165                 170                 175
```

```
Tyr Leu Gly Val Ser Asp Thr Pro Ala Trp Val Val Ser Ala Ala Asn
            180                 185                 190

Asn Tyr Ala Thr Ser His Gly Lys Thr Pro Phe Ser Ile Tyr Gln Gly
        195                 200                 205

Lys Trp Asn Val Leu Asn Arg Asp Phe Glu Arg Asp Ile Ile Pro Met
    210                 215                 220

Ala Arg His Phe Gly Met Ala Leu Ala Pro Trp Asp Val Met Gly Gly
225                 230                 235                 240

Gly Lys Phe Gln Ser Lys Lys Ala Met Glu Glu Trp Lys Lys Asn Gly
                245                 250                 255

Glu Gly Leu Arg Thr Ala Val Gly Gly Pro Glu Gln Thr Glu Leu Glu
            260                 265                 270

Val Lys Ile Ser Glu Ala Leu Asn Lys Ile Ala Glu Glu His Gly Thr
        275                 280                 285

Glu Ser Val Thr Ala Ile Ala Ile Ala Tyr Val Arg Ser Lys Ala Lys
    290                 295                 300

Asn Val Phe Pro Leu Val Gly Gly Arg Lys Ile Glu His Leu Lys Gln
305                 310                 315                 320

Asn Ile Glu Ala Leu Ser Ile Lys Leu Thr Pro Glu Gln Ile Glu Tyr
                325                 330                 335

Leu Glu Ser Ile Val Thr Phe Asp Val Gly Phe Pro Lys Ser Asn Ile
            340                 345                 350

Gly Asp Asp Pro Ala Val Thr Lys Lys Leu Ser Pro Leu Thr Ser Met
        355                 360                 365

Ser Ala Arg Ile Ser Phe Asp Asn
    370                 375
```

<210> 2
<211> 1131
<212> DNA
<213> artifical sequence

<400> 2

```
atgagcgatc tgtttaaacc ggcgccggaa ccgccgaccg aactgggccg cctgcgcgtg        60

ctgagcaaaa ccgcgggcat tcgcgtgagc ccgctgattc tgggcggcgc gagcattggc       120
```

gatgcgtgga gcggctttat gggcagcatg aacaaagaac aggcgtttga actgctggat 180

gcgttttatg aagcgggcgg caactgcgtg gataccgcga acagctatca gaacgaagaa 240

agcgaaattt ggattggcga atggatgaaa agccgcaaac tgcgcgatca gattgtgatt 300

gcgaccaaat ttaccggcga ttataaaaaa tatgaagtgg gcggcggcaa aagcgcgaac 360

tattgcggca accataaaca tagcctgcat gtgagcgtgc gcgatagcct gcgcaaactg 420

cagaccgatt ggattgatat tctgtatgtg cattggtggg attatatgag cagcattgaa 480

gaagtgatgg atagcctgca tattctgatt cagcagggca aagtgctgta tctgggcgtg 540

agcgataccc cggcgtgggt ggtgagcgcg gcgaacaact atgcgaccag ccatggcaaa 600

accccgttta gcatttatca gggcaaatgg aacgtgctga accgcgattt tgaacgcgat 660

attattccga tggcgcgcca ttttggcatg gcgctggcgc cgtgggatgt gatgggcggc 720

ggcaaatttc agagcaaaaa agcgatggaa gaatggaaaa aaaacggcga aggcctgcgc 780

accgcggtgg gcggcccgga acagaccgaa ctggaagtga aaattagcga agcgctgaac 840

aaaattgcgg aagaacatgg caccgaaagc gtgaccgcga ttgcgattgc gtatgtgcgc 900

agcaaagcga aaaacgtgtt tccgctggtg ggcggccgca aaattgaaca tctgaaacag 960

aacattgaag cgctgagcat taaactgacc ccggaacaga ttgaatatct ggaaagcatt 1020

gtgacctttg atgtgggctt tccgaaaagc aacattggcg atgatccggc ggtgaccaaa 1080

aaactgagcc cgctgaccag catgagcgcg cgcattagct ttgataacta a 1131

<210> 3
<211> 1131
<212> DNA
<213> artifical sequence

<400> 3
atgtctgatg tatttggacc tgcacctgaa ccacctaccg agttaggacg tctaagagtt 60

ctctctaaaa cagctggtat aagagtctct ccgctaatat tgggaggtat gtcgattggt 120

gacgcctggt caggattcat ggggtcaatg aacaaggagc gggcttttga gctgcttgat 180

gccttttttcg aggcaggtgg aaacttcatt gatactgcaa ataattacca aaatgaacag 240

tcagaggcat ggataggtga atggatggtt tcaagaaaat tgcgtgacca aattgttatt 300

gccaccaaat tcaccacaga ctataagaag tatgaagtgg gcaagggcag aagtgccaac 360

ttctgtggta atcacaagca tagtttacac gtaagtgtga gagattctct tcgcaaattg 420

cagactgatt ggattgacat tctctatgtt cactggtggg attatatgag ttcgatcgag 480

gaagttatgg atagtctgca tattcttgtg cagcagggca aggtcctcta cctgggagta 540

tctgatacac ctgcatgggt cgtgtctgct gcaaattact acgctacctc tcacgggaaa 600

actcccttca gcatctatca aggtaaatgg aatctgttga atagggactt tgagcgtgaa 660

```
attattccaa tggctaggca ttttggtatg gctctcgctc catgggatgt catgggaggg      720
ggaagatttc agagcaaaaa agctttagaa gaacggaaga agagtggaga gggcctgcgt      780
agctttgttg gtacatctga acagacggat gcagaggtta agatcagcga ggcattgtcg      840
aaggttgctg aggaacatgg cattgagtct gtcacagcta ttgccattgc ctatgtccgc      900
tccaaagcga agcatgtttt cccattggtt ggaggaagga aaattgagca cctcaaacaa      960
aatattgagg cgttgagtat taaattgaca ccaggacaga tagaatatct agaaagcatt     1020
gtcccattcg atgttgggtt ccctagcaat ttcatcggag atgatcctgc agttactaag     1080
aaacttgcat tccttccagc aatgtctgcc aagattgctt ttgacgatta g              1131
```